# EUROPEAN PATENT APPLICATION

(11) **EP 3 690 444 A1**
(43) Date of publication of application: **05.08.2020**
(21) Application number: 18861512.4
(22) Date of filing: 25.09.2018
(51) Int. Cl.: G01N 33/574, C07K 14/47, C12N 15/113, C12N 15/13, C12Q 1/02, C12Q 1/6804

(54) **METHOD FOR CONFIRMING PRDM14 EXPRESSION**

(30) Priority: 26.09.2017 JP 2017185200
(71) Applicant: The University Of Tokyo, Tokyo 113-8654 (JP); Kanagawa Prefectural Hospital Organization, Yokohama-shi, Kanagawa 231-0005 (JP)
(72) Inventor: IMAI, Kohzoh, Tokyo 113-8654 (JP); ZEMBUTSU, Hitoshi, Tokyo 135-8550 (JP); TANIGUCHI, Hiroaki, Tokyo 113-8654 (JP); SAITOH, Anri, Tokyo 113-8654 (JP); MIYAGI, Yohei, Yokohama-shi Kanagawa 241-8515 (JP)
(74) Representative: Hoffmann Eitle
(86) International application number: PCT/JP2018/035395
(87) International publication number: WO 2019/065609

(57) **Abstract**

One embodiment of this invention is a method for confirming expression of the PRDM14 gene from blood or lymphatic fluid collected from a subject by: (1) detecting PRDM14 positive CTC which expresses the PRDM14 gene; or (2) measuring the concentration of at least one protein selected from the group consisting of leptin receptor (LEPR), macrophage-derived chemokine (MDC) and gamma-interferon (IFNy).

SEQUENCE LISTING

<110>  THE UNIVERSITY OF TOKYO
       KANAGAWA PREFECTURAL HOSPITAL ORGANIZATION

<120>  A method of confirming the expression of PRDM14

<130>  PCT2828TKU

<140>  Not Yet Assigned
<141>  Concurrently Herewith

<150>  JP 2017-185200
<151>  2017-09-26

<160>  2

<170>  PatentIn version 3.5

<210>  1
<211>  571
<212>  PRT
<213>  Homo sapiens

<400>  1

Met Ala Leu Pro Arg Pro Ser Glu Ala Val Pro Gln Asp Lys Val Cys
1               5                   10                  15

Tyr Pro Pro Glu Ser Ser Pro Gln Asn Leu Ala Ala Tyr Tyr Thr Pro
            20                  25                  30

Phe Pro Ser Tyr Gly His Tyr Arg Asn Ser Leu Ala Thr Val Glu Glu
        35                  40                  45

Asp Phe Gln Pro Phe Arg Gln Leu Glu Ala Ala Ala Ser Ala Ala Pro
    50                  55                  60

Ala Met Pro Pro Phe Pro Phe Arg Met Ala Pro Pro Leu Leu Ser Pro
65                  70                  75                  80

Gly Leu Gly Leu Gln Arg Glu Pro Leu Tyr Asp Leu Pro Trp Tyr Ser
                85                  90                  95

Lys Leu Pro Pro Trp Tyr Pro Ile Pro His Val Pro Arg Glu Val Pro
            100                 105                 110

Pro Phe Leu Ser Ser Ser His Glu Tyr Ala Gly Ala Ser Ser Glu Asp
        115                 120                 125

Leu Gly His Gln Ile Ile Gly Gly Asp Asn Glu Ser Gly Pro Cys Cys
    130                 135                 140

Gly Pro Asp Thr Leu Ile Pro Pro Pro Pro Ala Asp Ala Ser Leu Leu
145                 150                 155                 160

Pro Glu Gly Leu Arg Thr Ser Gln Leu Leu Pro Cys Ser Pro Ser Lys
                165                 170                 175

Gln Ser Glu Asp Gly Pro Lys Pro Ser Asn Gln Glu Gly Lys Ser Pro
            180                 185                 190

Ala Arg Phe Gln Phe Thr Glu Glu Asp Leu His Phe Val Leu Tyr Gly

195                 200                 205

Val Thr Pro Ser Leu Glu His Pro Ala Ser Leu His His Ala Ile Ser
    210                 215                 220

Gly Leu Leu Val Pro Pro Asp Ser Ser Gly Ser Asp Ser Leu Pro Gln
225                 230                 235                 240

Thr Leu Asp Lys Asp Ser Leu Gln Leu Pro Glu Gly Leu Cys Leu Met
                245                 250                 255

Gln Thr Val Phe Gly Glu Val Pro His Phe Gly Val Phe Cys Ser Ser
            260                 265                 270

Phe Ile Ala Lys Gly Val Arg Phe Gly Pro Phe Gln Gly Lys Val Val
        275                 280                 285

Asn Ala Ser Glu Val Lys Thr Tyr Gly Asp Asn Ser Val Met Trp Glu
    290                 295                 300

Ile Phe Glu Asp Gly His Leu Ser His Phe Ile Asp Gly Lys Gly Gly
305                 310                 315                 320

Thr Gly Asn Trp Met Ser Tyr Val Asn Cys Ala Arg Phe Pro Lys Glu
                325                 330                 335

Gln Asn Leu Val Ala Val Gln Cys Gln Gly His Ile Phe Tyr Glu Ser
            340                 345                 350

Cys Lys Glu Ile His Gln Asn Gln Glu Leu Leu Val Trp Tyr Gly Asp
        355                 360                 365

Cys Tyr Glu Lys Phe Leu Asp Ile Pro Val Ser Leu Gln Val Thr Glu
    370                 375                 380

Pro Gly Lys Gln Pro Ser Gly Pro Ser Glu Glu Ser Ala Glu Gly Tyr
385                 390                 395                 400

Arg Cys Glu Arg Cys Gly Lys Val Phe Thr Tyr Lys Tyr Tyr Arg Asp
                405                 410                 415

Lys His Leu Lys Tyr Thr Pro Cys Val Asp Lys Gly Asp Arg Lys Phe
            420                 425                 430

Pro Cys Ser Leu Cys Lys Arg Ser Phe Glu Lys Arg Asp Arg Leu Arg
        435                 440                 445

Ile His Ile Leu His Val His Glu Lys His Arg Pro His Lys Cys Ser
    450                 455                 460

Thr Cys Gly Lys Cys Phe Ser Gln Ser Ser Ser Leu Asn Lys His Met
465                 470                 475                 480

Arg Val His Ser Gly Asp Arg Pro Tyr Gln Cys Val Tyr Cys Thr Lys
                485                 490                 495

Arg Phe Thr Ala Ser Ser Ile Leu Arg Thr His Ile Arg Gln His Ser
            500                 505                 510

Gly Glu Lys Pro Phe Lys Cys Lys Tyr Cys Gly Lys Ser Phe Ala Ser
        515                 520                 525

His Ala Ala His Asp Ser His Val Arg Arg Ser His Lys Glu Asp Asp
    530                 535                 540

Gly Cys Ser Cys Ser Ile Cys Gly Lys Ile Phe Ser Asp Gln Glu Thr
545                 550                 555                 560

Phe Tyr Ser His Met Lys Phe His Glu Asp Tyr
                565                 570

<210>  2
<211>  2344
<212>  DNA
<213>  Homo sapiens

<400>  2
aattccctac cctcgacctg tcgatgcccc gcggccccgc ccgccctctt aagcctggct      60

cagccctcag ggcccgcccg aagtctaccg agcccgagtg gcctaccgag cccgagtggc     120

cccgcagcgt ccaggaggcg cccgctccgc ggtggcgctc ttggaggtgg tgtcggagag     180

ccgccgagcg tgcggtcccg ggatggctct accccggcca agtgaggccg tgcctcagga     240

caaggtgtgc tacccgccgg agagcagccc gcagaacctg gccgcgtact acacgccttt     300

cccgtcctat ggacactaca gaaacagcct ggccaccgtg gaggaagact tccaaccttt     360

ccggcagctg gaggccgcag cgtctgctgc ccccgccatg ccccccttcc ccttccggat     420

ggcgcctccc ttgctgagcc cgggtctggg cctacagagg gagcctctct acgatctgcc     480

ctggtacagc aagctgccac cgtggtaccc aattccccac gtccccaggg aagtgccgcc     540

cttcctgagc agcagccacg agtacgcggg tgccagcagt gaagatctgg gccaccaaat     600

cattggtggc gacaacgaga gtggcccgtg ttgtggacct gacactttaa ttccaccgcc     660

ccctgcggat gcttctctgt tacctgaggg gctgaggacc tcccagttat taccttgctc     720

acccagcaag cagtcagagg atggtcccaa accctccaac caagaaggga agtcccctgc     780

tcggttccag ttcacggagg aggacctgca cttcgttctg tacggggtca ctcccagcct     840

ggagcaccca gccagcctgc accatgcgat ttcaggcctc ctggtccccc cagacagctc     900

tggatctgat tctcttcctc aaactctgga taaagactcc cttcaacttc cagaaggtct     960

atgcctcatg cagacggtgt ttggtgaagt cccacatttt ggtgtgttct gcagtagttt    1020

tatcgccaaa ggagtcaggt ttgggccctt tcaaggtaaa gtggtcaatg ccagtgaagt    1080

gaagacctac ggagacaatt ctgtgatgtg ggagatcttt gaagatggtc atttgagcca    1140

ctttatagat ggaaaaggag gtacggggaa ctggatgtcc tatgtcaact gtgcccgctt    1200

ccccaaggag cagaacctag ttgctgtgca gtgtcaaggg catatatttt atgagagctg    1260

caaagagatc catcagaacc aagagctcct tgtgtggtat ggagactgct atgagaaatt    1320

tctggatatt cctgtgagcc ttcaggtcac agagccgggg aagcagccat ctgggccctc    1380

tgaagagtct gcagaaggct acagatgtga aagatgtggg aaggtattta cctacaaata 1440

ttacagagat aagcacctca agtacacccc ctgtgtggac aagggcgata ggaaatttcc 1500

ctgttctctc tgcaaacgat cctttgagaa gcgggaccgg cttcggatcc acattcttca 1560

tgttcatgag aagcaccggc ctcacaagtg ttctacatgt gggaaatgtt tctctcaatc 1620

ttccagccta aacaaacaca tgcgagtcca ctctggagac agaccatacc agtgtgtgta 1680

ttgtactaag aggttcacag cctccagcat actccgcaca cacatcaggc agcactccgg 1740

ggagaagccc ttcaaatgca agtactgtgg taaatctttt gcatcccatg ctgcccatga 1800

cagccatgtc cggcgttcac acaaggagga tgatggctgc tcatgcagca tctgtgggaa 1860

aatcttctca gatcaagaaa cattctactc ccacatgaag tttcatgaag actactagcc 1920

ctgccaggca caatgactca cgcctgtaat cccagcactt tgggaggcag aggtgggtgg 1980

atcactcaag tccaggagtt cgagaccagc ctgggcaaca tggtgaaatc ctgtctctac 2040

caaaaaaata caaaaatcag ctgggggtgg tggcacatgc ctgtggttcc agccactcag 2100

gaggtcgagg tggcaggatg gtttgagcac aggagacgga ggttgctgtg agctgagatc 2160

gccccactgc ttttcaacct gggtgacaga accagaccct gtctcaaaac aaaacaaaac 2220

aaaaaaaatg agtagccctc aagagtgtgg agacaatgta aaaacaagag attcggattc 2280

tctctatttc cttttatggg ttatagaagt ccctgcagtt ggctgtgtgt ggtggctcac 2340

gcct 2344

## Description

### [TECHNICAL FIELD]

The present invention relates to a method for confirming expression of PRDM14 gene from the blood or lymphatic fluid collected from a subject, and more specifically, relates to (1) a peripheral blood circulating tumor cell biomarker for use in the confirmation method, a diagnostic method of cancer, a method for judging efficacy and prognosis of a therapeutic agent for cancer, a kit comprising a detection reagent for the biomarker, a method for identifying peripheral blood circulating tumor cells, as well as a method for diagnosing a disease involving PRDM14 gene expression, a method for evaluating efficacy of a therapeutic agent for the disease, a method for determining a therapeutic policy for the disease, and/or a method for judging prognosis after therapy of the disease, and a cancer metastasis marker and a kit that can be used in those methods.

### [BACKGROUND ART]

PRDM (PR domain-containing protein) 14 has been identified as a nuclear transcription factor that is transiently expressed in ES cells (embryonic stem cells) and primordial germ cells, and it is composed of a PR domain with high homology to SET domain and six Zn finger domains. Expression of PRDM14 molecules is accelerated in a tumor-site localized manner in breast cancer, pancreatic cancer, non-small cell lung cancer and the like, but the expression is not observed in adult normal tissues (Non-Patent Documents 1 to 3). PRDM14 gene products as a biomarker for these cancers are not a secretory protein or a cell membrane protein, and they are present in the nucleus; therefore, it is difficult to handle them as a tumor marker that can be easily detected from blood.

### [PRIOR ART DOCUMENTS]

### [NON-PATENT DOCUMENT]

[Non-patent Document 1] Nishikawa N., et al., Cancer Res. (2007) 67(20) pp. 9649-9657
[Non-Patent Document 2] Taniguchi H., et al., Oncotarget (2017) 8(29) pp. 46856-46874
[Non-Patent Document 3] Moriya C., et al., Carcinogenesis (2017) 38(6) pp. 638-648

### [DISCLOSURE OF INVENTION]

### [PROBLEMS TO BE SOLVED BY THE INVENTION]

The present invention aims to provide a method and a biomarker for minimally invasive detection of breast cancer, pancreatic cancer, or non-small cell lung cancer from a subject, a method for diagnosing a disease involving PRDM14 gene expression, a method for evaluating efficacy of a therapeutic agent targeting PRDM14 for the disease, a method for determining a therapeutic policy for the disease, and/or a method for judging prognosis after therapy of the disease.

### [MEANS FOR SOLVING THE PROBLEMS]

In order to solve the above-mentioned problems, the present inventors have continued intensive studies, and as factors correlated with PRDM14 gene expression, the present inventors have focused on the concentrations of three secretory proteins, leptin receptor (LEPR), macrophage-derived chemokine (MDC) and interferon gamma (IFNy), in blood or lymphatic fluid, as well as peripheral blood circulating tumor cells CTCs. CTC is known as a tumor cell released from a primary tumor tissue or a metastatic tumor tissue and infiltrated into the blood, and it is present in a minute amount in the peripheral blood of a patient with solid cancer.

In recent years, a CTC test for measuring the number of cancer cells in peripheral blood has attracted attention. Meanwhile, the present inventors have found that PRDM14-positive CTCs can be detected by staining specific molecules in CTCs isolated from the blood of a subject; and in addition, found that, by measuring concentrations of these secretory proteins in a sample and comparing them with the reference concentration range specified by the present inventors for the first time, it is possible, for example, to diagnose a disease involving PRDM14 gene expression such as breast cancer, to evaluate the efficacy of a therapeutic agent targeting PRDM14 for the disease, to determine a therapeutic policy of the disease, and/or to judge the prognosis after therapy of the disease; and thus, the present inventors have completed the present invention.

Namely, the present invention relates to the following.
[1] A method for confirming the expression of PRDM14 (PR domain-containing protein 14) gene in the blood or lymphatic fluid collected from a subject, by:
   (1) detecting a peripheral blood circulating tumor cell that expresses PRDM14 gene (PRDM14-positive CTC), or
   (2) measuring the concentration of at least one protein selected from the group consisting of leptin receptor (LEPR), macrophage-derived chemokine (MDC) and interferon gamma (IFNy).
[2] A biomarker for use in the confirmation method (1) according to [1], comprising a PRDM14-positive CTC.
[3] The biomarker according to [2], wherein the PRDM14-positive CTC is a cancer stem cell.
[4] The biomarker according to [2] or [3] for diagnosing at least one kind of cancer selected from the group consisting of breast cancer, pancreatic cancer and non-small cell lung cancer.
[5] The biomarker according to [4] for judging that an inhibitor of PRDM14 gene expression selected from antisense, siRNA or shRNA that inhibits the expression of PRDM14 gene expression is effective as a therapeutic agent for cancer.
[6] The biomarker according to [4] for judging that an anti-PRDM14 antibody or a fragment thereof is effective as a therapeutic agent for cancer, wherein the antibody and its fragment bind to a PRDM14 protein.
[7] The biomarker according to [5] or [6] for determining a therapeutic policy for cancer of administering to a subject a pharmaceutical composition containing an effective amount of an inhibitor of PRDM14 gene expression or an anti-PRDM14 antibody or a fragment thereof.
[8] The biomarker according to [4] for judging that the prognosis is poor.
[9] In cases when the biomarker according to [2] or [3] is detected from a subject,
   a method for assisting the diagnosis that the subject is suffering from at least one cancer selected from the group consisting of breast cancer, pancreatic cancer and non-small cell lung cancer, or that the subject has suffered from the cancer.
[10] In cases when the biomarker according to [4] is detected from a subject,
   a method for judging that an inhibitor of PRDM14 gene expression selected from antisense, siRNA or shRNA that inhibits the expression of PRDM14 gene, or an anti-PRDM14 antibody or a fragment thereof is effective as a therapeutic agent for cancer,
   wherein the antibody and the fragment thereof bind to a PRDM14 protein.
[11] In cases when the biomarker according to [5] or [6] is detected from a subject,
   a method for determining a therapeutic policy for cancer of administering to the subject a pharmaceutical composition containing an effective amount of an inhibitor of PRDM14 gene expression or an anti-PRDM14 antibody or a fragment thereof.
[12] In cases when the biomarker according to [4] is detected from a subject, a method for judging that the prognosis is poor.
[13] A kit for determining the presence or absence of PRDM14-positive CTCs, the kit comprising a reagent for detecting the biomarker according to any one of [2] to [8].
[14] The kit according to [13], wherein the reagent is a reagent for staining PRDM14 protein, cytokeratin, CD45 and cell nucleus, respectively.
[15] A method for detecting PRDM14-positive CTCs in the blood collected from a subject, the method comprising the following steps:
   (i) a step of collecting a band containing peripheral blood mononuclear cells (PBMCs) from the blood by density gradient centrifugation;
   (ii) a step of isolating EpCAM-positive cells from the band using an anti-EpCAM (CD326) antibody; and
   (iii) a step of staining PRDM14 protein, cytokeratin, CD45 and cell nucleus in the EpCAM-positive cells;
   wherein in step (iii), EpCAM cells in which PRDM14 protein, cytokeratin and cell nucleus are stained but CD45 is not stained are identified as PRDM14-positive CTCs.
[16] A method for:
   (A) assisting diagnosis of a disease involving PRDM14 gene expression,
   (B) evaluating efficacy of a therapeutic agent for the disease targeting PRDM14,
   (C) assisting determination of a therapeutic policy for the disease, and/or,
   (D) assisting judgement of prognosis after therapy of the disease,
   in a subject, wherein the method comprises the following steps:
   (i) a step of performing the confirmation method (2) according to [1]; and
   (ii) in cases when at least one of the obtained concentrations satisfies the reference concentration range, the following steps:
      in (A), assisting the diagnosis that the subject has the disease,
      in (B), evaluating that the therapeutic agent is effective for the subject having the disease,
      in (C), assisting the determination of the therapeutic policy of administering the therapeutic agent to the subject,
      in (D), assisting the judgement that the prognosis after the therapy of the disease is not good.
[17] The method according to [16], wherein the disease is breast cancer or pancreatic cancer.
[18] The method according to [16] or [17], wherein the therapeutic agent contains an inhibitor of PRDM14 gene expression selected from antisense, siRNA or shRNA that inhibits PRDM14 gene expression.
[19] The method according to [16] or [17], wherein the therapeutic agent comprises an anti-PRDM14 antibody or a fragment thereof, and the antibody and the fragment thereof bind to a PRDM14 protein.
[20] In cases when the sample is blood, the method according to any one of [16] to [19], wherein,
   the reference concentration range of LEPR is 4.3 ng/ml or more;
   the reference concentration range of MDC is 11.2 pg/ml or less, or,
   the reference concentration range of IFNy is 3.2 pg/ml or less.
[21] The method according to [18] or [19], wherein the therapeutic agent is used for companion diagnostics for molecular targeted therapy of cancer.
[22] A cancer metastasis marker for use in the method according to [20], comprising at least one protein selected from the group consisting of LEPR, MDC and IFNy.
[23] A kit for use in the method according to any one of [16] to [20], comprising an antibody or a fragment thereof that binds to at least one protein selected from the group consisting of LEPR, MDC and IFNγ.

### [ADVANTAGEOUS EFFECTS OF INVENTION]

When the biomarker for use in the confirmation method (1) according to the present invention is detected, the presence or presence of cancer such as breast cancer and pancreatic cancer can be easily specified with minimal invasion to the subject.

Furthermore, according to the confirmation method (2) of the present invention, diagnosis of a disease involving PRDM14 gene expression can be easily performed in a simple manner from a sample of minimal invasion such as blood, without directly collecting a target diseased tissue; in addition, by evaluating the efficacy of a therapeutic agent for a disease targeting PRDM14, the disease can be treated without applying unnecessary therapeutic agents to the subject. It is possible to determine a therapeutic policy for the disease, that is, administering a PRDM14-targeted therapeutic agent, and to judge the prognosis after therapy of the disease. Moreover, cancer metastasis markers and kits that can be used in these methods can also be provided.

The present inventors attempted to detect CTCs in 36 cases of breast cancer patients, and found that CTCs were detected from 29 cases (80%), and that 19 cases (about 65%) among them were positive for PRDM14 (Example 1); therefore, when the biomarker according to the present invention is used, it is possible to judge that whether the PRDM14-targeted therapeutic agent is effective or not, whether the administration of such a therapeutic agent is appropriate as a therapeutic policy or not, and whether the prognosis after therapy is poor or not. Thereby, for example, among breast cancer patients, PRDM14-positive or PRDM14-negative breast cancer patients are distinguished, so that more accurate therapy can be applied to each patient.

### [BRIEF DESCRIPTION OF DRAWINGS]

[FIG. 1] Figure 1 is a schematic diagram showing one embodiment of the method for identifying a PRDM14-positive CTC according to the present invention.
[FIG. 2] Figure 2 is a graph comparing the amount of tumor marker CA15-3 contained in the plasma collected from breast cancer patients and the number of CTCs for each patient.
[FIG. 3] Figure 3 is a graph comparing the amount of CA15-3 contained in the plasma collected from breast cancer patients and the PRDM14 positive rate of CTCs for each patient.
[FIG. 4] Figure 4 is a diagram in which the number of CTCs and the PRDM14 positive rate of CTCs are plotted against the amount of CA15-3 contained in the plasma collected from breast cancer patients.
[FIG. 5-1] Figure 5-1 shows micrographs of CTCs isolated from the blood of pancreatic cancer patients.
[FIG. 5-2] Figure 5-2 shows micrographs of PRDM14-positive CTCs isolated from the blood of breast cancer patients and pancreatic cancer patients.
[FIG. 6] Figure 6 is a schematic diagram outlining a series of screening methods for identifying serum biomarkers that correlate with the expression level of PRDM14 gene.
[FIG. 7] Figure 7 shows the results of an experiment in which the correlation between the PRDM14 gene expression level and, for example, the LEPR gene expression level was verified.
[FIG. 8] Figure 8 shows box-and-whisker plots showing statistically significant differences in the LEPR, MDC, and IFNy identified as serum biomarkers that correlate with PRDM14 gene expression levels, between PRDM14 high expression group and low expression group. The upper and lower limits of the box and the horizontal line inside the box indicate the 75th and 25th percentile values and the median, respectively. The upper and lower horizontal bars represent the 90th and 10th percentile values, respectively.
[FIG. 9] Figure 9 is a diagram outlining indices of the efficacy of a PRDM14-targeted therapeutic agent and a method for evaluating the efficacy.

### [BEST MODE FOR CARRYING OUT THE INVENTION]

Hereinafter, the present invention will be described in detail.

Unless otherwise indicated herein, scientific and technical terms used in connection with the present invention shall have the meanings that are commonly understood by those of ordinary skill in the art. In general, the terms and techniques used with respect to cells and tissue cultures described in the present specification, and with respect to molecular biology, immunology, microbiology, gene and protein and nucleic acid chemistry, shall be those that are well known and commonly used in the art. All patents, patent applications and other publications referred to herein are incorporated herein by reference in their entirety.

Also, unless otherwise indicated, the methods and techniques of the present invention will be performed in accordance with conventional methods well known in the art, and as described in various general and more specialized reference documents that are cited and discussed in the present specification. Examples of such documents include Sambrook et al., Molecular Cloning: A Laboratory Manual, 2nd ed., Cold Spring Harbor Laboratory Press (1989) and Sambrook et al., Molecular Cloning: A Laboratory Manual, 3rd ed., Cold Spring Harbor Press (2001); Ausubel et al., Current Protocols in Molecular Biology, Greene Publishing Associates (Appendix in 1992 and 2000); Ausubel et al., Short Protocols in Molecular Biology: A Compendium of Methods from Current Protocols in Molecular Biology-4th Ed., Wiley & Sons (1999); Harlow and Lane, Antibodies: A Laboratory Manual, Cold Spring Harbor Laboratory Press (1990); and Harlow and Lane, Using Antibodies: A Laboratory Manual, Cold Spring Harbor Laboratory Press (1999), *etc.*

The terms used herein for analytical chemistry, synthetic organic chemistry and medicinal chemistry and pharmaceutical chemistry, as well as experimental procedures and techniques thereof, are those that are well known and commonly used in the art. Standard techniques shall be used for chemical synthesis, chemical analysis, and manufacture, formulation and delivery of drugs, and treatment of a subject.

In the present specification, unless otherwise specified, the name of a protein is represented by alphabetical capital letters or katakana, and the gene encoding the protein is represented by the above-mentioned alphabetical capital letters or katakana plus "gene" or by the underlined above-mentioned alphabetical capital letters or katakana. Therefore, unless otherwise specified, for example, when simply described as "PRDM14", it means a protein that is PRDM14 (PR domain-containing protein 14) itself, and when described as "PRDM14 gene", it means a gene encoding PRDM14; and the expression "PRDM14" also means a gene encoding PRDM14.

As described above, PRDM14 used in the present invention is a protein having a PR domain and a zinc finger domain, which is called PR domain-containing protein 14 (also known as PFM11 or MGC59730), and the examples include proteins containing an amino acid sequence identical or substantially identical to the amino acid sequence represented by SEQ ID NO: 1. Here, an example of the amino acid sequence substantially identical to the amino acid sequence represented by SEQ ID NO: 1 is an amino acid sequence having a homology of about 70% or more, preferably about 80% or more, more preferably about 90% or more, still more preferably about 95% or more, and most preferably about 98% or more to the amino acid sequence represented by SEQ ID NO: 1.

In addition, the PRDM14 gene used in the present invention include, a DNA containing a base sequence represented by SEQ ID NO: 2, or, a DNA containing a base sequence that specifically hybridizes to the base sequence represented by SEQ ID NO: 2 under stringent conditions and encoding a protein having substantially the same properties as the protein containing the amino acid sequence represented by SEQ ID NO: 1. Here, as a DNA containing a base sequence that specifically hybridizes under stringent conditions to the base sequence represented by SEQ ID NO: 2, for example, a DNA containing the base sequence having a homology of about 60% or more, preferably about 70% or more, more preferably about 80% or more, still more preferably about 90% or more, particularly preferably about 95% or more, and most preferably about 98% or more to the base sequence represented by SEQ ID NO: 2, can be used. Here, the "stringent conditions" are, usually, conditions at 42°C, 2xSSC and 0.1% SDS, and preferably, at 65°C, 0.1×SSC and 0.1% SDS.

In the present specification, the term "cancer" is also used to mean "malignant tumor" which is not limited to "carcinoma".

The base sequence of the PRDM14 gene may be different depending on polymorphism, isoform and others, not only between different species of animals, but also between the same species of animals; however, even when base sequences are different, they are included in the PRDM14 gene as far as they encode PRDM14.

In the present invention, "gene expression" refers to a series of biological reactions starting from transcription of the gene to translation. In quantifying the expression level of mRNA encoding PRDM14, known techniques, such as hybridization techniques (northern hybridization, dot hybridization, RNase protection assay, cDNA microarray, *etc*.), gene amplification techniques (reverse transcription polymerase chain reaction) (RT-PCR) (including competitive RT-PCR, real-time PCR, *etc*.)) and the like may be utilized. When using a hybridization technique, a polynucleotide encoding PRDM14 or an oligonucleotide or polynucleotide capable of hybridizing to a part thereof can be used as a probe; when using a gene amplification technique, such oligonucleotide or polynucleotide can be used as a primer. "Polynucleotide encoding PRDM14 or a part thereof" includes both DNA and RNA, and includes, for example, mRNA, cDNA, cRNA and the like. Therefore, the nucleotide constituting the oligonucleotide or polynucleotide may be either deoxyribonucleotide or ribonucleotide. In quantifying the expression level of mRNA encoding PRDM14 in the present invention, the base length of the oligonucleotide used is not particularly limited, but is usually 15 to 100 bases, preferably 17 to 35 bases; and the base length of the polynucleotide used is not particularly limited, but is usually 50 to 1000 bases, preferably 150 to 500 bases.

In quantifying the expression level of PRDM14, a known technique, a known protein analysis technique, for example, western blotting method based on an antigen-antibody reaction using an anti-PRDM14 antibody or a fragment thereof, a dot blot method, an immunoprecipitation method, an ELISA, an immunohistochemistry (IHC) or the like can be used. "Significantly higher gene expression" means that the expression level of mRNA and/or the expression level of a polypeptide including a protein is statistically significantly higher than that of a control, or, is preferably at least 1.5 times, more preferably at least 2 times, even more preferably at least 3 times, and most preferably at least 5 times higher. The expression level of the PRDM14 gene is preferably corrected based on the expression level of a gene whose expression level does not largely fluctuate between tissues or individuals (*e.g*., housekeeping genes such as β-actin gene and GAPDH gene) as an internal standard gene.

In addition, when described as "LEPR", "MDC" and "IFNγ"; it means a protein itself, *i.e*., a leptin receptor, a macrophage-derived chemokine and interferon-gamma, respectively, and when described as "LEPR gene", "MDC gene" and "IFNγ gene", it means a gene encoding LEPR, a gene encoding MDC, and a gene encoding IFNγ, respectively. Furthermore, when described as "LEPR", "MDC" and "IFNy", it also means a gene encoding LEPR, MDC and IFNγ, respectively.

Examples of the LEPR, MDC and IFNy used in the present invention include proteins containing an amino acid sequence that is the same or substantially the same as the amino acid sequences represented by SEQ ID NOs: 1, 2 and 3. Here, as an amino acid sequence substantially the same as the amino acid sequence represented by each of SEQ ID NOs: 1, 2, and 3, an amino acid sequence having a homology of about 70% or more, preferably about 80% or more, more preferably about 90% or more, still more preferably about 95% or more, and most preferably about 98% or more to the amino acid sequence represented by each of SEQ ID NOs: 1, 2, and 3 can be exemplified.

The present invention provides a method for confirming the expression of PRDM14 gene in the blood or lymphatic fluid collected from a subject, by: (1) detecting a peripheral blood circulating tumor cell expressing PRDM14 gene (PRDM14-positive CTC), or (2) measuring the concentration of at least one protein selected from the group consisting of leptin receptor (LEPR), macrophage-derived chemokine (MDC) and interferon gamma (IFNγ) (also simply referred to as confirmation method). The present inventors have also searched for members other than PRDM14 of the PRDM family, cancer markers, and stem cell markers, which can be used in place of PRDM14 for diagnosing cancer, *etc*. in the present invention; however, the one which shows better efficacy than PRDM14 has not yet been found.

### <Biomarker>

The present invention also provides a biomarker comprising PRDM14-positive CTCs for use in the confirmation method (1).

PRDM14-positive CTCs may be a cancer stem cell. "Cancer stem cell" refers to a cell having the characteristics of a stem cell among cancer cells, and "stem cell" refers to a cell that maintains differentiation potency even after cell division. When cancer stem cells are stained with Hoechst fluorescent dye (Hoechst33342) and detected by flow cytometry using a UV laser (wavelength of about 350 nm) as excitation light, they are concentrated in a side population (SP) fraction. The SP fraction is a fraction that is not stained due to the discharge of a dye out of the cell via an ABC transporter or the like, with respect to main population (MP) fractions stained with the Hoechst fluorescent dye. In addition, stem cells can also be identified by marker molecules such as Oct3/4, Nanog, SSEA1, and SSEA4.

The present invention also provides, in one embodiment, a biomarker for diagnosing at least one cancer selected from the group consisting of breast cancer, pancreatic cancer and non-small cell lung cancer; and in another embodiment, in cases when the biomarker is detected from a subject, the present invention provides a method for diagnosing that the subject is suffering from the cancer or has suffered from the cancer, or a method for assisting such diagnosis.

As used herein, the term "subject" refers to any living individual, preferably a vertebrate, more preferably a mammal, for example, a rodent such as mouse, rat, gerbil, or guinea pig, *etc*., a cat such as cat, puma, or tiger, *etc*., a deer such as deer and elk, *etc*., as well as a rabbit, a dog, a mink, a sheep, a goat, a cow, a horse, a monkey, a human, *etc*., and particularly preferably it means a human. In yet another embodiment, a human can also be excluded from the subject. The subject may be healthy or suffer from any disease, but when treatment for a disease is contemplated, it typically means a subject suffering from the disease or being at a risk of suffering from the disease.

Furthermore, in one embodiment, the present invention provides a biomarker used for judging that an inhibitor of PRDM14 gene expression selected from antisense, siRNA or shRNA that inhibits PRDM14 gene expression is effective as a therapeutic agent for cancer; and in another embodiment, in cases when the above-mentioned biomarker is detected from a subject, the present invention provides a method for judging that the above inhibitor of PRDM14 gene expression is effective as a therapeutic agent for cancer. In addition, in one embodiment, the present invention provides the above-mentioned biomarker for judging that an anti-PRDM14 antibody or a fragment thereof is effective as a therapeutic agent for cancer; and in another embodiment, in cases when the above-mentioned biomarker is detected from a subject, the present invention provides a method for judging that the anti-PRDM14 antibody or a fragment thereof is effective as a therapeutic agent for cancer. In this specification, the "inhibitor of PRDM14 gene expression" and the "anti-PRDM14 antibody or a fragment thereof" may be collectively referred to as "PRDM14 targeted therapeutic agents". In the present invention, the anti-PRDM14 antibody may be a monoclonal antibody or a polyclonal antibody, and a fragment of such an antibody can also bind to the PRDM14 protein to the same extent as the anti-PRDM14 antibody. As the PRDM14 targeted therapeutic agent, those described in JP No. 5219818 can be used without limitation.

In one embodiment, the present invention provides the above-mentioned biomarker for determining a therapeutic policy for cancer of administering to a subject a pharmaceutical composition comprising an effective amount of an inhibitor of PRDM14 gene expression or an anti-PRDM14 antibody or a fragment thereof; and in another embodiment, in cases when the above-mentioned biomarker is detected from a subject, the present invention provides a method for determining a therapeutic policy for cancer of administering the pharmaceutical composition to the subject. In a further embodiment, the present invention can determine the expected therapeutic effect by comparing the number of PRDM14-positive CTCs in the blood before start of the chemotherapy and after the end of each cycle when one or more cycles have been completed after starting such chemotherapy.

In some embodiments, the present invention provides the above-mentioned biomarker for judging that prognosis after cancer therapy is poor, and in other embodiments, in cases when the above-mentioned biomarker is detected from a subject, the present invention provides a method for judging that the prognosis after cancer therapy is poor or a method for assisting such judgment.

### <CTC identification method/kit>

The present invention provides a method for detecting PRDM14-positive CTCs in the blood collected from a subject, comprising the following steps (i) to (iii), wherein in step (iii), EpCAM cells in which PRDM14 protein, cytokeratin and cell nucleus are stained but CD45 is not stained are identified as PRDM14-positive CTCs.
(i) A step of collecting a band containing peripheral blood mononuclear cells (PBMCs) from the blood by density gradient centrifugation.
(ii) A step of isolating EpCAM-positive cells from the band using an anti-EpCAM (CD326) antibody.
(iii) A step of staining PRDM14 protein, cytokeratin, CD45 and cell nucleus in the EpCAM-positive cells.

### Step (i):

Step (i) is a step of collecting a band containing peripheral blood mononuclear cells (PBMCs) from the blood collected from a subject by density gradient centrifugation.

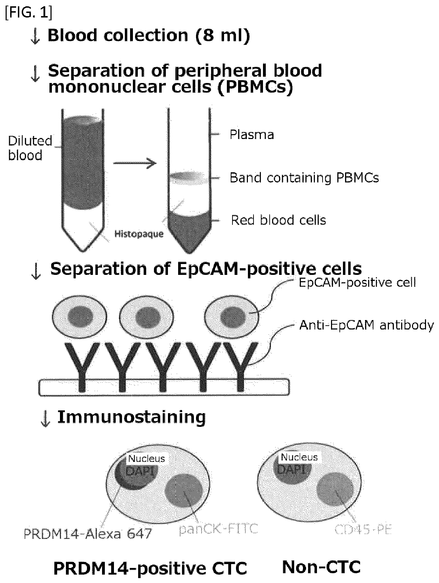

The density gradient centrifugation method and the density gradient centrifugation medium, respectively, are not particularly limited as long as they are commonly performed as blood cell separation in the art. In one embodiment, as shown in FIG. 1, the collected blood is appropriately diluted, and when density gradient centrifugation using Histopaque®-1077 (10771, manufactured by SIGMA-ALDRICH) as a density gradient centrifugation medium is performed, then PBMCs form a band (or a layer or buffy coat) at the interface between plasma and red blood cells (Histopaque).

The band that contains PBMCs can also contain T cells, B cells, monocytes, granulocytes, dendritic cells, and the like, as well as CTCs when the subject is suffering from cancer, or has suffered from cancer.

Step (ii):
Step (ii) is a step of isolating EpCAM-positive cells from the band collected in step (i) using an anti-EpCAM (epithelial cell adhesion molecule) antibody or an anti-CD326 antibody.
In one embodiment, when the collected band is resuspended and loaded onto CytoQuest™ CR system of Abnova, EpCAM-positive cells are captured by EpCAM (KA4439, Abnova) that is immobilized on the CytoChipNano (U0095, Abnova). Even when CTCs are present, CTCs are contained with a number of only about several to several tens per 10 mL of blood, so that step (ii) has the effect of enriching EpCAM-positive cells.

Step (iii):
Step (iii) is a step of staining PRDM14 protein, cytokeratin, CD45 and cell nuclei in the EpCAM-positive cells isolated in step (ii).
For example, for staining PRDM14 protein, an anti-PRDM14 antibody labeled with a red fluorescent dye such as Alexa Fluor® 647, *etc*. can be used; for staining cytokeratin (CK), an anti-cytokeratin antibody (e.g.*,* an anti-PanCK antibody) labeled with a green fluorescent dye such as FITC, *etc*. can be used; for staining CD45, an anti-CD45 (leukocyte common antigen LCA) antibody labeled with an orange fluorescent dye such as PE (R-phycoerythrin), *etc.* can be used; for staining cell nuclei, a blue fluorescent dye DAPI (4',6-diamidino-2-phenylindole) that strongly binds to DNA, *etc*. can be used.
Regarding the immunostaining that may be performed in step (iii), a procedure usually performed in the art can be employed.

As a result of step (iii), it can be identified as follows:
- Those that are DAPI negative and CD45 negative are cell debris and dust, *etc.*
- DAPI-positive and CD45-positive cells are non-cancer PBMCs.
- DAPI-positive and CD45-negative cells are CTCs,
   and, when the cells are cytokeratin positive and PRDM14 positive, they can be identified as PRDM14-positive CTCs.

The present invention also provides, in one embodiment, a kit for determining the presence or absence of PRDM14-positive CTCs from the blood of a subject, wherein the kit comprises a reagent for detecting the above-mentioned biomarker; and in further embodiment, as the reagent, reagents for staining PRDM14 protein, cytokeratin, CD45, and cell nuclei, respectively, can be comprised. Furthermore, the kit of the present invention may comprise CytoChipNano (U0095, Abnova) or CytoQuest™ CR (Abnova), and may also comprise instructions for using the kit.

### <Diagnostic method/Efficacy evaluation method/Therapeutic policy determination method/Prognosis judgment method>

### The present invention relates to, in a subject,

(A) a method of assisting diagnosis of a disease involving PRDM14 gene expression (herein also simply referred to as "diagnostic method"),
(B) a method for evaluating efficacy of a therapeutic agent for the disease targeting PRDM14 (herein also simply referred to as "efficacy evaluation method"),
(C) a method for assisting determination of a therapeutic policy for the disease (herein also simply referred to as "therapeutic policy determination method"), and/or
(D) a method for assisting judgement of prognosis after therapy of the disease (herein also simply referred to as "prognosis judgment method"),
   wherein each of these methods comprises the following steps (i) and (ii):
   (i) a step of performing the confirmation method (2) according to the present invention, that is, a step of measuring the concentration of at least one protein selected from the group consisting of LEPR, MDC and IFNy from the blood or lymphatic fluid collected from the subject,
   (ii) in cases when at least one of the obtained concentrations satisfies the reference concentration range, the following steps:
      in (A), diagnosing that the subject has the disease,
      in (B), evaluating that the therapeutic agent is effective for the subject having the disease,
      in (C), determining the therapeutic policy of administering the therapeutic agent to the subject,
      in (D), assisting the judgement that the prognosis after the therapy of the disease is not good.

The "disease involving PRDM14 gene expression" is a disease that comprises a lesion in which PRDM14 gene expression (excluding transient PRDM14 gene expression in ES cells (embryonic stem cells) or primordial germ cells) is observed, and preferably it is cancer, more preferably breast cancer, pancreatic cancer, or non-small cell lung cancer with poor differentiation.

As used herein, the term "diagnosis" includes, for example, identifying or classifying a disease involving PRDM14 gene expression and its stage. One embodiment of diagnosing is, a protein in a sample collected from a subject is measured and in cases when the obtained concentration satisfies a reference concentration range, a method of assisting diagnosis that the subject has the disease, or a method for such diagnosis.

As used herein, the term "therapeutic agent" may be any drug or agent for treating a disease, including, but not limited to, a medicament, a pharmaceutical composition, a therapeutic agent, an internal agent, an external agent and the like, as well as a therapeutic agent for cancer, and further including a therapeutic agent for breast cancer or pancreatic cancer. One embodiment of the therapeutic agent is an inhibitor of PRDM14 gene expression selected from antisense, siRNA, or shRNA that inhibits PRDM14 gene expression. In certain embodiments, such therapeutic agents comprise an anti-PRDM14 antibody or a fragment thereof, wherein said fragment of the antibody binds to the PRDM14 protein to the same extent as the antibody. In the present specification, these therapeutic agents targeting PRDM14 are also simply referred to as "PRDM14 targeted therapeutic agents", and those described in JP No. 5219818 can be used without limitation.

In yet another embodiment, such therapeutic agents may be used for companion diagnostics for molecular targeted therapy of cancer. In addition, the cancer which is the subject of the cancer molecularly targeted therapy may include not only cancers included in the above-mentioned diseases involving PRDM14 gene expression, but also cancers metastasized from the cancers involving PRDM14 gene expression (however, metastatic cancers do not always involve PRDM14 gene expression). Such metastatic cancers include, but are not limited to, for example, cancers of the lung, bone, brain, *etc.* in which breast cancer is likely to metastasize, and cancers of the liver, peritoneum, *etc.* in which pancreatic cancer is likely to metastasize.

As used herein, the term "treatment" includes therapy, and further includes all types of medically acceptable prophylactic and/or therapeutic intervention for the purpose of curing, temporarily ameliorating, or preventing a disease. In addition, "treatment" includes medically acceptable intervention for various purposes, including delaying or stopping the progression of a disease, regression or elimination of a lesion, prevention of onset or prevention of recurrence of the disease, and the like.

As used herein, the term "evaluating efficacy" refers to judging whether or not to contribute to the cure, temporary remission or prevention of a disease, or to have such an effect; when the contribution or the effect is present, it means effective. One embodiment of evaluating efficacy is a method of evaluation in which a protein in a sample collected from a subject is measured, and when the obtained concentration satisfies a reference concentration range, the therapeutic agent is evaluated to be effective for the subject having a disease.

As used herein, the term "determination of therapeutic policy" means to determine the direction of a treatable means to be performed in the hope of cure, temporary remission or prevention of a disease. One embodiment of determining a therapeutic policy is, a protein in a sample collected from a subject is measured and in cases when the obtained concentration satisfies a reference concentration range, a method for assisting determination of a therapeutic policy of administering a therapeutic agent to the subject, or a method for determining such a therapeutic policy.

As used herein, the term "judgement of prognosis after therapy" means predicting whether or not an effect such as cure, temporary remission or prevention of a disease is exhibited. One embodiment of judging the prognosis after therapy is, a protein in a sample collected from a subject is measured and in cases when the obtained concentration satisfies a reference concentration range, a method for assisting judgement that the prognosis after therapy of the disease is not good, or a method for such judgement.

In the present specification, the term "judgement of not good" means to judge that cure, temporary remission or prevention of a disease cannot be expected, or there is no such effect.

The "sample collected from a subject" is preferably blood or lymphatic fluid, and more preferably blood, but is not particularly limited as long as it is a body fluid having the same or almost the same composition as serum.

The protein to be measured in a sample is preferably a secretory protein, more preferably LEPR, MDC and IFNγ, *etc.* The number of proteins to be measured is at least one, and preferably a plurality (for example, two or three). In one embodiment of the present invention, the protein to be measured may be, for example, at least one protein selected from the group consisting of LEPR, MDC and IFNγ, and further, at least two proteins selected from the group consisting of LEPR, MDC and IFNγ, and moreover, each of the proteins of LEPR, MDC and IFNy may be measured.

In addition, as a result of measuring the concentration of one of these three proteins, if the score is 1 with reference to the following reference concentration range, the diagnosis, efficacy evaluation, determination of therapeutic policy, and judgement of prognosis according to the present invention can be performed. In this case, it is not necessary to measure the concentration of the other two types, but if a measurement is performed and the results show a score of 2 or 3, then the credibility of the diagnosis, efficacy evaluation, determination of therapeutic policy, and judgement of prognosis may become high.

The reference concentration range of the measured protein is, for example, 4.3 ng/mL or more for LEPR, 11.2 pg/mL or less for MDC, or 3.2 pg/mL or less for IFNγ. Furthermore, another embodiment of the present invention includes the following: after separating and scoring measured values using a cut-off value of the above-mentioned reference concentration, the values are further summed up and may be used in the method for assisting diagnosis, the method for evaluating the efficacy, and the method for assisting determination of a therapeutic policy, or a method for assisting judgement of prognosis, *etc*., of the present invention. In particular embodiments of the invention, for example, cut-off values of LEPR are ≤4.3 ng/ml, >4.3 ng/ml, and cut-off values of MDC are <11.2 pg/ml, ≥11.2 pg/ml, and cut-off values of IFNy are <3.2 pg/ml, ≥3.2 pg/ml, *etc*. In addition, for example, when the measured value of LEPR is ≤4.3 ng/ml, the score is set to 0, and when the measured value of LEPR is >4.3 ng/ml, the score is set to 1; when the measured value of MDC is <11.2 pg/ml, the score is set to 1, and when it is ≥11.2 pg/ml, the score is set to 0; when the measured value of IFNy is <3.2 pg/ml, the score is set to 1, and when it is ≥3.2 pg/ml, the score is set to 0; then the values of the respective scores are summed and can be used in the present invention. When the total score is 1 or more, (A) the subject can be diagnosed that he/she is suffering from a disease involving PRDM14 gene expression, (B) the PRDM14 targeted therapeutic agent can be evaluated to be effective, and (C) as a therapeutic policy for the disease, administration of a PRDM14 targeted therapeutic agent can be determined, and/or (D) a poor prognosis after therapy of the disease can be judged. When the score is 0, the result is that all of the items in (A) to (D) are "incapable".

As a method for measuring the concentration of a protein, any method known in the art, preferably a method such as ELISA can be used.

### <Cancer metastasis marker>

In addition, another aspect of the present invention relates to a cancer metastasis marker comprising at least one protein selected from the group consisting of LEPR, MDC and IFNγ, for use in the above-mentioned diagnostic method, efficacy evaluation method, therapeutic policy determination method, and/or prognosis judgment method.

### <Kit>

Another embodiment of the present invention relates to a kit for use in the above-mentioned diagnostic method, efficacy evaluation method, therapeutic policy determination method and/or prognosis judgement method, wherein the kit comprises an antibody or a fragment thereof that binds to at least one protein selected from the group consisting of LEPR, MDC and IFNγ.

In one embodiment, the kit of the present invention may comprise the following without limitation: a reagent containing an antibody or a fragment thereof that binds to a protein such as LEPR, MDC and IFNγ(for example, a LEPR detection reagent, etc.), instruments (for example, pipette, dropper, tweezers, *etc*.), instructions on the method to assist diagnosis, the method to evaluate efficacy, the method to assist determination of therapeutic policy, or the method to assist judgement of prognosis (*e.g.* instructions for use, an medium recording the information on how to use, for example, flexible disk, CD, DVD, Blu-ray disk, memory card, USB memory, *etc*.).

The following experimental examples explain the present invention more specifically, and they do not at all limit the scope of the present invention. Those skilled in the art having ordinary knowledge and techniques can make various modifications to the embodiments shown in the following experimental examples, without departing from the spirit of the present invention, and such modified embodiments are also included in the present invention.

### [EXAMPLES]

### <EXAMPLE 1>

In the method for detecting PRDM14-positive CTCs according to the present invention, CTCs were detected from 36 specimens of 2-mL blood collected from breast cancer patients and the number thereof was measured, in accordance with the instruction attached to CytoQuest® CR of Abnova, and in addition, the PRDM14 positive rate was calculated by immunostaining using an anti-PRDM14 antibody (product codes: ab187881 and ab192411; Abcam). Table 1 shows the results.

### [TABLE 1]

**Table 1: Number of CTCs and PRDM14 positive rate in breast cancer specimens**

| Patient No. | **Number of CTCs** | **PRDM14 positive rate (%)** | **Patient No.** | **Number of CTCs** | **PRDM14 positive rate (%)** |
|---|---|---|---|---|---|
| C26 | 7 | 40 | C63 | 552 | 0 |
| C28 | 55 | 100 | C64 | 162 | 0 |
| C30 | 0 | - | C65 | 128 | 37.5 |
| C31 | 5 | 40 | C68 | 240 | 0 |
| C32 | 0 | - | C71 | 40 | 0 |
| C33 | 0 | - | C72 | 120 | 0 |
| C35 | 0 | - | C74 | 4 | 0 |
| C36 | 16 | 66.7 | C75 | 608 | 26.3 |
| C37 | 0 | - | C76 | 80 | 0 |
| C38 | 140 | 100 | C77 | 0 | - |
| C39 | 7 | 28.6 | C78 | 440 | 45.5 |
| C40 | 20 | 10 | C79 | 232 | 34.5 |
| C41 | 49 | 0 | C82 | 344 | 72.1 |
| C51 | 175 | 90.9 | C83 | 232 | 20.7 |
| C52 | 79 | 61.5 | C84 | 920 | 45.7 |
| C58 | 0 | - | C85 | 4 | 0 |
| C59 | 912 | 0 | C87 | 128 | 7.7 |
| C60 | 792 | 10 | C88 | 125 | 26.7 |

Of all 36 specimens listed in Table 1, one or more CTCs were present in 29 specimens (80.6%), of which 19 were PRDM14 positive (65.5%) and 10 were PRDM14 negative (34.4%). Although there appears to be no correlation between the number of CTCs and the PRDM14 positive rate (Table 1), about two-thirds of the specimens in which CTC detection was possible were found to be PRDM14 positive.

### <EXAMPLE 2>

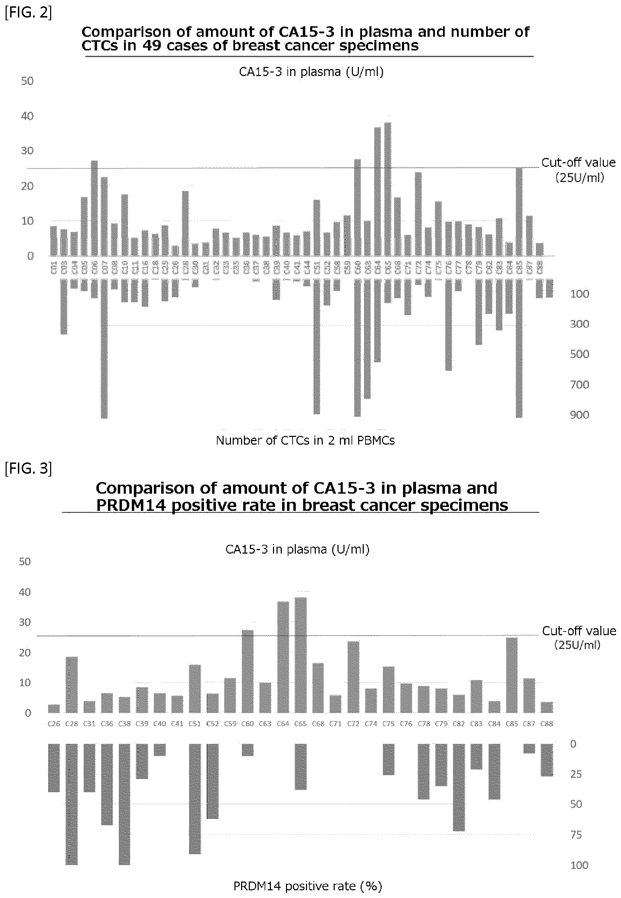

Using PRDM14 and the tumor marker CA15-3 which is considered useful for tracking recurrence and metastasis of breast cancer, the amount of CA15-3 in plasma was measured while measuring the number of CTCs as in Example 1, and the PRDM14 positive rate was calculated. Results are shown in FIGs. 2 to 4, respectively.

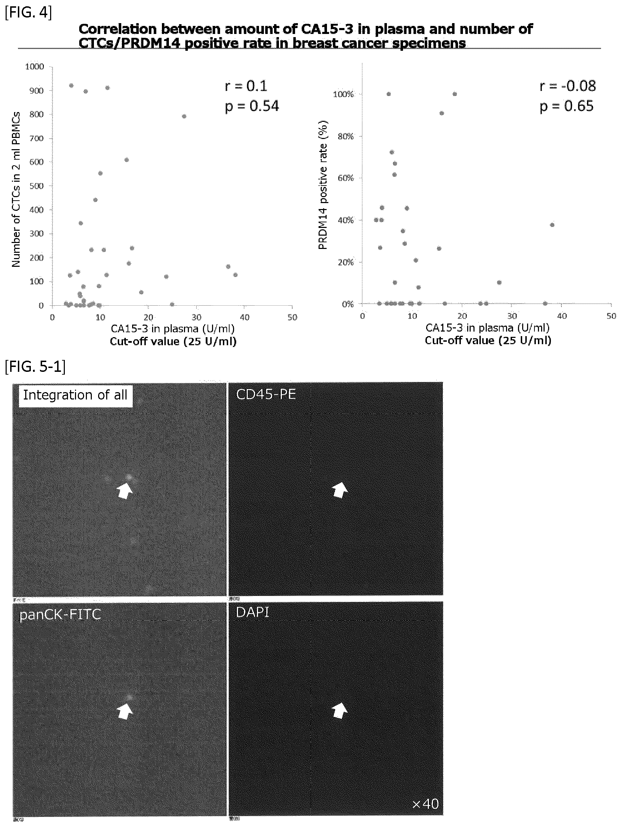

There was no correlation between the amount of CA15-3 in plasma, and the number of CTCs and the PRDM14 positive rate, respectively (FIG. 4), but the number of specimens that were PRDM14 positive and CTC detectable were greater than that of CA15-3-positive specimens.

The following shows the stage of breast cancer, number of CTCs and PRDM14 positive rate.

### [TABLE 2]

**Table 2: Stage of disease, number of CTCs and PRDM14 positive rate.**

| Patient No. | **Clinical stage** | **Pathological stage** | **Pre-surgical hormonal therapy** | **Pre-surgical chemotherapy** | **Number of CTCs** | **PRDM14 positive rate (%)** |
|---|---|---|---|---|---|---|
| C26 | I | IIA | | | 5 | 40 |
| C28 | IIA | IIB | None | None | 55 | 100 |
| C30 | I | | | | 0 | 0 |
| C31 | I | | | | 5 | 40 |
| C32 | IIA | | | | 0 | 0 |
| C33 | I | | | | 0 | 0 |
| C36 | I | | | | 16 | 67 |
| C37 | I | | | | 0 | 0 |
| C38 | I | | | | 140 | 100 |
| C39 | I | | | | 7 | 29 |
| C40 | IIIB | | | | 20 | 10 |
| C41 | IIA | | | | 49 | 0 |
| C51 | IIA | | | | 175 | 91 |
| C52 | I | | | | 79 | 62 |

Although CA15-3 is rarely positive in early breast cancer, Table 2 suggests that unlike conventional tumor markers, PRDM14 can be used for diagnosis even in early breast cancer patients including stage I.

### <REFERENCE EXAMPLE>

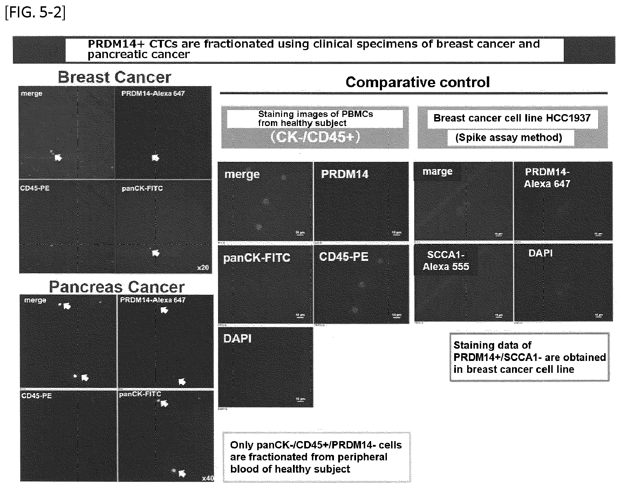

Figure 5-1 shows microscopic images of CTCs isolated from peripheral blood of a pancreatic cancer patient by CytoQuest® CR (Abnova), and FIG. 5-2 shows microscopic images of PRDM14-positive CTCs fractionated from clinical specimens of breast cancer and pancreatic cancer.

### <EXAMPLE 3>

### <<Identification of biomarkers that correlate with PRDM14 gene expression level>>

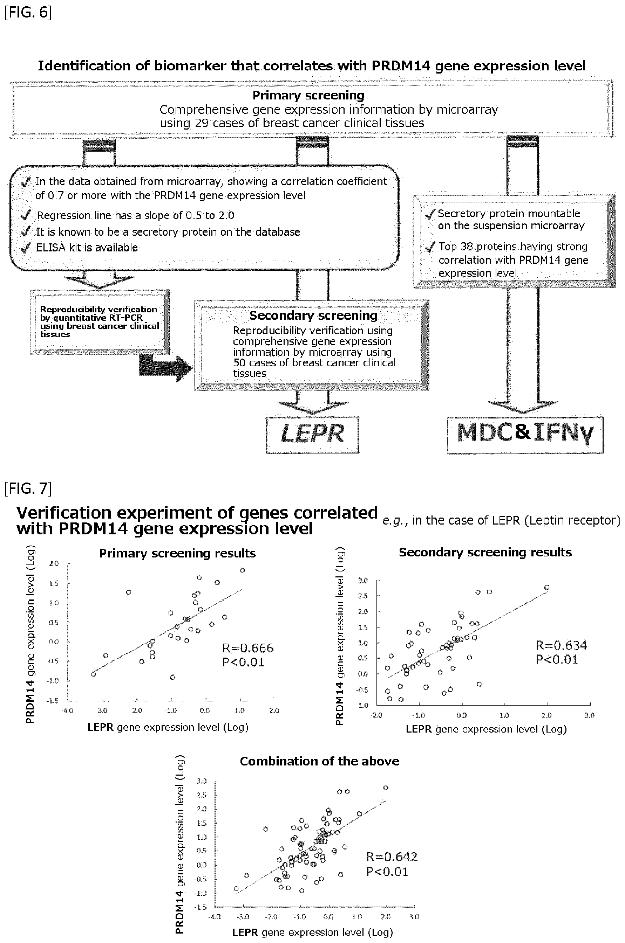

Figure 6 schematically outlines a series of screening methods for identifying a biomarker that correlates with the expression level of PRDM14 gene. First, in the primary screening, comprehensive gene expression information by microarray in 29 cases of breast cancer clinical tissues was analyzed, and candidate molecules were narrowed down based on the following conditions: a correlation with the expression level of PRDM14 gene found to be frequently amplified (about 60%) in breast cancer (1) with a correlation coefficient of 0.7 or more, (2) the regression line has a slope of 0.5 to 2.0, and (3) it is known to be a secretory protein on the database, (4) ELISA kit is available. Then, in the secondary screening, reproducibility verification using comprehensive gene expression information by microarray for another 50 cases of breast cancer clinical tissues was performed, and it was found that LEPR correlated with the expression level of PRDM14 gene.

In addition, after the primary screening, from another viewpoint, that is, among the secretory proteins that can be mounted on a suspension array, i.e., a serum protein screening tool, the top 38 proteins having a strong correlation with the PRDM14 expression level were examined, and MDC and IFNy were found to be correlated with the expression level of PRDM14 gene.

Next, a verification experiment of the PRDM14 gene expression level and the LEPR gene expression level was performed (FIG. 7). In the verification test, the correlation between the PRDM14 gene expression level and the LEPR gene expression level was examined based on the above screening results, with the vertical axis representing the PRDM14 gene expression level and the horizontal axis representing the LEPR gene expression level. In FIG. 7, the upper left graph shows the result of the primary screening, the upper right graph shows the result of the secondary screening, and the lower graph corresponds to their combined graph. As a result, it was found that the PRDM14 gene expression level and the LEPR gene expression level have a positive correlation.

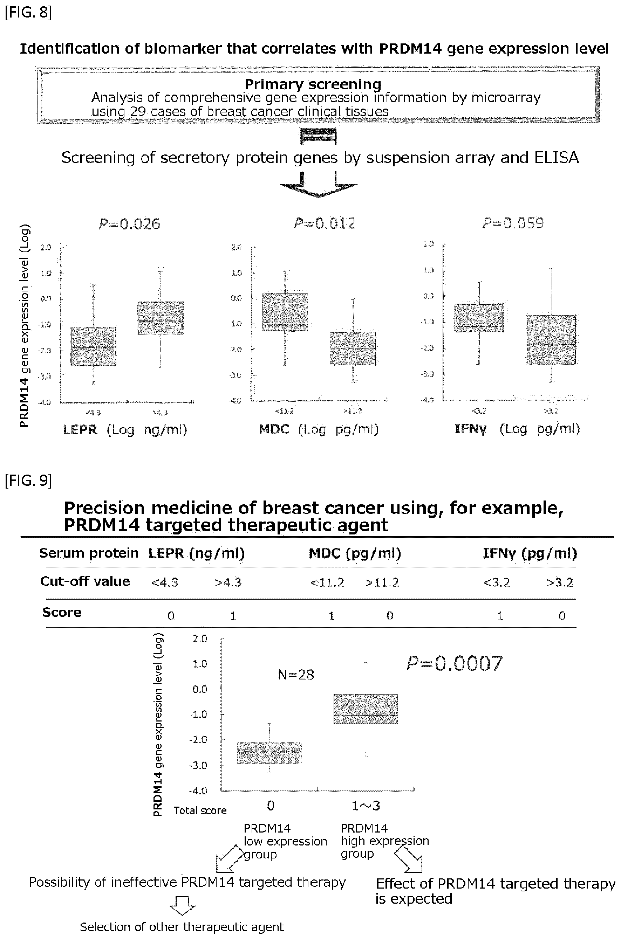

Furthermore, the correlation between the PRDM14 gene expression level and the blood concentration of MDC or IFNy was examined in the same manner as described above. As a result, screening of secretory protein genes by suspension array and ELISA was performed after the primary screening, and it was found that the PRDM14 gene expression level and the blood LEPR concentration have a positive correlation, and that the PRDM14 gene expression level and the blood concentrations of MDC and IFNy have negative correlations (FIG. 8). That is, when the amount of LEPR secreted into the blood is large, it can be understood that the PRDM14 gene is highly expressed, and also, when the secreted amounts of MDC and IFNγ are both small, it can be understood that the PRDM14 gene is highly expressed.

The various features described in this specification can be variously combined, and the embodiments obtained by such combinations are all within the scope of the present invention, including the combinations not specifically described in this specification. In addition, those skilled in the art understand that many various modifications are possible without departing from the spirit of the present invention, and equivalents comprising such modifications are also included in the scope of the present invention. Therefore, it is to be understood that the embodiments described herein are illustrative only, and they are not described with the intention of limiting the scope of the present invention.

### [INDUSTRIAL APPLICABILITY]

When the biomarker according to the present invention is detected from a subject, it is specified that cancer such as breast cancer or pancreatic cancer is present or has been present, and it is possible to judge that a PRDM14 targeted therapeutic agent is effective, to determine a therapeutic policy of administering a pharmaceutical composition containing an effective amount of a PRDM14 targeted therapeutic agent, and to judge that prognosis after therapy is poor. Furthermore, for example among breast cancer patients, PRDM14-positive patients or PRDM14-negative patients are distinguished, so that more accurate therapy can be applied to each types of patients.

In addition, according to one embodiment of the present invention, efficacy of a PRDM14 targeted therapeutic agent can be evaluated; for example, since not all subjects suffering from breast cancer express the PRDM14 gene, before administering a PRDM14 targeted therapeutic agent, it is possible to judge whether the PRDM14 target therapy is effective or ineffective for the subject.

## Claims

1. A method for confirming the expression of PRDM14 (PR domain-containing protein 14) gene in the blood or lymphatic fluid collected from a subject, by:
(1) detecting a peripheral blood circulating tumor cell that expresses PRDM14 gene (PRDM14-positive CTC), or
(2) measuring the concentration of at least one protein selected from the group consisting of leptin receptor (LEPR), macrophage-derived chemokine (MDC) and interferon gamma (IFNy).

2. A biomarker for use in the confirmation method (1) according to claim 1, comprising a PRDM14-positive CTC.

3. The biomarker according to claim 2, wherein the PRDM14-positive CTC is a cancer stem cell.

4. The biomarker according to claim 2 or 3 for diagnosing at least one kind of cancer selected from the group consisting of breast cancer, pancreatic cancer and non-small cell lung cancer.

5. The biomarker according to claim 4 for judging that an inhibitor of PRDM14 gene expression selected from antisense, siRNA or shRNA that inhibits the expression of PRDM14 gene expression is effective as a therapeutic agent for cancer.

6. The biomarker according to claim 4 for judging that an anti-PRDM14 antibody or a fragment thereof is effective as a therapeutic agent for cancer, wherein the antibody and its fragment bind to a PRDM14 protein.

7. The biomarker according to claim 5 or 6 for determining a therapeutic policy for cancer of administering to a subject a pharmaceutical composition containing an effective amount of an inhibitor of PRDM14 gene expression or an anti-PRDM14 antibody or a fragment thereof.

8. The biomarker according to claim 4 for judging that the prognosis is poor.

9. In cases when the biomarker according to claim 2 or 3 is detected from a subject,
a method for assisting the diagnosis that the subject is suffering from at least one cancer selected from the group consisting of breast cancer, pancreatic cancer and non-small cell lung cancer, or that the subject has suffered from the cancer.

10. In cases when the biomarker according to claim 4 is detected from a subject,
a method for judging that an inhibitor of PRDM14 gene expression selected from antisense, siRNA or shRNA that inhibits the expression of PRDM14 gene, or an anti-PRDM14 antibody or a fragment thereof is effective as a therapeutic agent for cancer,
wherein the antibody and the fragment thereof bind to a PRDM14 protein.

11. In cases when the biomarker according to claim 5 or 6 is detected from a subject,
a method for determining a therapeutic policy for cancer of administering to the subject a pharmaceutical composition containing an effective amount of an inhibitor of PRDM14 gene expression or an anti-PRDM14 antibody or a fragment thereof.

12. In cases when the biomarker according to claim 4 is detected from a subject,
a method for judging that the prognosis is poor.

13. A kit for determining the presence or absence of PRDM14-positive CTCs, the kit comprising a reagent for detecting the biomarker according to any one of claims 2 to 8.

14. The kit according to claim 13, wherein the reagent is a reagent for staining PRDM14 protein, cytokeratin, CD45 and cell nucleus, respectively.

15. A method for detecting PRDM14-positive CTCs in the blood collected from a subject, the method comprising the following steps:
(i) a step of collecting a band containing peripheral blood mononuclear cells (PBMCs) from the blood by density gradient centrifugation;
(ii) a step of isolating EpCAM-positive cells from the band using an anti-EpCAM (CD326) antibody; and
(iii) a step of staining PRDM14 protein, cytokeratin, CD45 and cell nucleus in the EpCAM-positive cells;
wherein in step (iii), EpCAM cells in which PRDM14 protein, cytokeratin and cell nucleus are stained but CD45 is not stained are identified as PRDM14-positive CTCs.

16. A method for:
(A) assisting diagnosis of a disease involving PRDM14 gene expression,
(B) evaluating efficacy of a therapeutic agent for the disease targeting PRDM14,
(C) assisting determination of a therapeutic policy for the disease, and/or,
(D) assisting judgement of prognosis after therapy of the disease, in a subject, wherein the method comprises the following steps:
(i) a step of performing the confirmation method (2) according to claim 1; and
(ii) in cases when at least one of the obtained concentrations satisfies the reference concentration range, the following steps:
in (A), assisting the diagnosis that the subject has the disease,
in (B), evaluating that the therapeutic agent is effective for the subject having the disease,
in (C), assisting the determination of the therapeutic policy of administering the therapeutic agent to the subject,
in (D), assisting the judgement that the prognosis after the therapy of the disease is not good.

17. The method according to claim 16, wherein the disease is breast cancer or pancreatic cancer.

18. The method according to claim 16 or 17, wherein the therapeutic agent contains an inhibitor of PRDM14 gene expression selected from antisense, siRNA or shRNA that inhibits PRDM14 gene expression.

19. The method according to claim 16 or 17, wherein the therapeutic agent comprises an anti-PRDM14 antibody or a fragment thereof, and the antibody and the fragment thereof bind to a PRDM14 protein.

20. In cases when the sample is blood, the method according to any one of claims 16 to 19, wherein,
the reference concentration range of LEPR is 4.3 ng/ml or more;
the reference concentration range of MDC is 11.2 pg/ml or less, or,
the reference concentration range of IFNy is 3.2 pg/ml or less.

21. The method according to claim 18 or 19, wherein the therapeutic agent is used for companion diagnostics for molecular targeted therapy of cancer.

22. A cancer metastasis marker for use in the method according to claim 20, comprising at least one protein selected from the group consisting of LEPR, MDC and IFNγ.

23. A kit for use in the method according to any one of claims 16 to 20, comprising an antibody or a fragment thereof that binds to at least one protein selected from the group consisting of LEPR, MDC and IFNγ.
